(19) Europäisches Patentamt
European Patent Office
Office européen des brevets

(11) Publication number: **0 074 792**
**B1**

(12) **EUROPEAN PATENT SPECIFICATION**

(45) Date of publication of patent specification: **27.11.85**

(51) Int. Cl.$^4$: **C 07 C 61/40,** C 07 C 69/743, C 07 C 51/487, C 07 C 67/60

(21) Application number: **82304711.3**

(22) Date of filing: **08.09.82**

(54) **Isomer enrichment process for cyclopropane carboxylates.**

(30) Priority: **16.09.81 US 302796**

(43) Date of publication of application:
**23.03.83 Bulletin 83/12**

(45) Publication of the grant of the patent:
**27.11.85 Bulletin 85/48**

(84) Designated Contracting States:
**BE CH DE FR GB IT LI NL**

(56) References cited:
**EP-A-0 034 875**
**FR-A-2 206 310**

(73) Proprietor: **ICI AMERICAS INC**
**Concord Pike & New Murphy Road**
**Wilmington Delaware 19897 (US)**

(72) Inventor: **Hartmann, Ludwig Albert**
**2211 Nassau Drive**
**Wilmington Delaware 19810 (US)**

(74) Representative: **Bishop, Nigel Douglas et al**
**Imperial Chemical Industries PLC Legal Department: Patents PO Box 6**
**Welwyn Garden City Herts, AL7 1HD (GB)**

**EP 0 074 792 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Natural and synthetic cyclopropane derivatives have been of considerable interest in the pesticide field. In particular, various esters or analogues of chrysanthemic or pyrethric acids have achieved commercial acceptance as insecticides on a wide scale. Specific examples of such compounds are those of the following Formula (I):

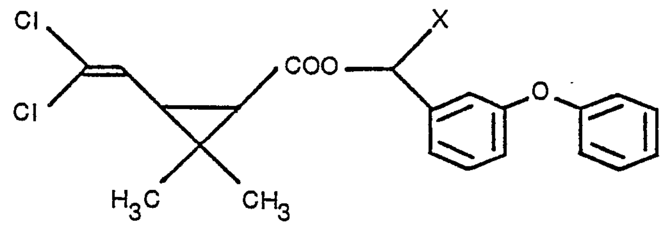

(I)

wherein X may be hydrogen to define the structure of permethrin and cyano to define cypermethrin as described in U.S. Patent 4,024,163. Insecticidal compounds of the Formula (I) may be prepared by esterification of a compound of the following Formula (II) with a compound of the following Formula (III):

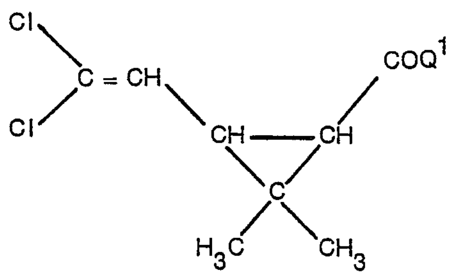

(II)

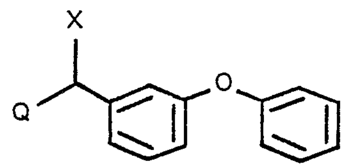

(III)

wherein Q and $COQ^1$ are functional groups or atoms which will react together to form an ester linkage and X is as described for Formula (I). It is usually convenient to react the acid or acid halide with the alcohol, e.g. $COQ^1$ is COOH or COHalide and Q is OH or to react a halogeno compound, i.e., Q is halogen and X=H, with a salt of the carboxylic acid, i.e., $COQ^1$ is $COO^-M^+$ where M is, for example, a silver or triethylammonium cation as set forth in U.S. Patent 4,024,163. Further, the acid chloride of Formula (II), i.e., wherein $COQ^1$ is COHalide may be reacted with the aldehyde derivative of Formula (III), i.e., wherein Q is =O and X is H, in the presence of a cyanide to yield the pesticide of Formula (I) wherein X is cyano. Such reactions are described in U.S. Patents 4,024,163, 4,254,050, 4,254,051, 4,254,052 and 4,280,965. Carboxylic acid or acid chloride compounds of Formula (II) may be obtained from the corresponding intermediate ester, e.g. $COQ^1$ is $COOH^3$, by hydrolysis with strong base or hydrolysis followed by halogenation, respectively. In turn, the intermediate esters may be prepared as described in "Recent Advances in Synthetic Pyrethroids" by Alfred Bader in Aldrichimica Acta *9,* No. 3, pages 49—51 (1976).

It is known that various pyrethroid type cyclopropane derivatives exhibit a difference in biological activity depending on whether the cis or trans isomer is used as set forth in U.S. Patent 4,024,163. Interconversions between cis and trans in this series are known but involve considerable chemical effort and expense, see Elliot and James in "Pyrethrum, the Natural Insecticide" by John E. Casida, Academic Press at Page 76 (1973).

An object of the present invention is a process which adjusts the cis/trans ratio of a vicinal-substituted cyclopropane carboxylate compound or changes the ratio of a product of a reaction of a vicinal-substituted cyclopropane carboxylate compared to the starting material. The vicinal-substituted cyclopropane carboxylates or said reaction products are known intermediates in the synthesis of pyrethroid pesticides.

The present invention provides a method of adjusting the cis/trans ratio of a vicinally-disubstituted cyclopropane, a carboxylate being one of the substituents, by the use of a controlled hydrolysis reaction of the carboxylate functionality whereby less than 100% of the starting material is converted to the corresponding acid or salt. It has been found that the cis and trans isomers do not hydrolyse at the same rate and this factor may be used to recover the carboxylate and/or the product acid in a different cis/trans ratio than was present in the starting material.

The starting material for the process of the invention is a cis/trans isomeric mixture of a vicinal-substituted cyclopropane carboxylic acid ester or a mixture of different vicinal-substituted cyclopropane carboxylates. Of particular interest are compounds of the following Formula (IV):

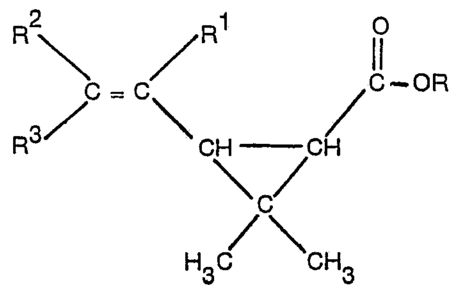

(IV)

wherein $R^1$ represents hydrogen; $R^2$ and $R^3$, which may be the same or different, represent halogen, e.g., fluorine, chlorine, bromine or iodine, or a halogenated alkyl group, e.g., a lower alkyl group of about 1 to 6 carbons having at least one halogen atom such as fluorine, chlorine, bromine or iodine, an example being trifluoromethyl; and R may be an alkyl group such as a lower alkyl group of about 1 to 6 carbons, e.g., a methyl or ethyl group, or an aryl group such as a phenyl group or a benzyl or substituted benzyl group. Particular examples of compounds of Formula (IV) are the 2,2-dimethyl-3-(2,2-dichlorovinyl)cyclopropane carboxylates and 2,2-dimethyl-3-(2-chloro-3,3,3-trifluoroprop-1-en-1-yl) cyclopropane carboxylates and particular carboxylates are the methyl and ethyl esters.

As used in the present invention, cis and trans refer to the relationship between two substituents on vicinal, i.e., adjacent, carbons of a cyclopropane ring. An example of this usage is the vinyl and ester moieties directly attached to the cyclopropane ring in Formula (IV), see Chapter 7 of "Stereochemistry of Carbon Compounds" by E. L. Eliel, McGraw-Hill, New York (1962).

A first embodiment of the present invention involves the steps of i) partially hydrolysing the vicinal-substituted cyclopropane carboxylate to form less than 100 mole % of the corresponding acid or salt thereof: ii) separating the vicinal-substituted cyclopropane carboxylic acid formed in step i) from the unreacted vicinal-substituted cyclopropane carboxylate; and iii) recovering the cis-enriched vicinal-substituted cyclopropane carboxylate. In a second embodiment of the invention, steps i) and ii) of the first embodiment are carried out but the third step is the recovery of the trans-enriched vicinal-substituted carboxylic acid reaction product. If a trans-enriched vicinal-substituted carboxylic acid ester is desired, the trans-enriched carboxylic acid may simply be esterified with an alkanol by conventional means. The trans-enriched carboxylate may be used as an intermediate for a pyrethroid final product having a high trans content. A high trans material may be less active as a pesticide than a cis-enriched pyrethroid and lower activity may be desirable in view of a lessened effect against non-target species. Alternatively, the trans-enriched material may be accumulated and reprocessed to raise the cis content, e.g., by equilibration.

The first step of either embodiment of the invention involves hydrolysis of less than 100 mole % of the vicinal-substituted cyclopropane carboxylate, more particularly about 30 to 70 mole %. The partial hydrolysis may be accomplished by acidic or basic hydrolysis. In particular, basic hydrolysis may be carried out utilizing a base such as an alkali metal hydroxide, e.g., NaOH or KOH, or an alkaline earth metal hydroxide or carbonate in less than 100 mole % or by using a base at a lower hydrolysis temperature or for a shorter reaction time. Variation in these parameters, i.e., the particular base, the molar amount of the base, reaction temperature and reaction time, may be used to control the rate and extent of hydrolysis. The hydrolysis may be carried out at a temperature less than about 100°C, e.g., from about 20°C to 100°C. High temperature may result in degradation of the starting material. One or more solvents may be present during the hydrolysis reaction. Particular solvents for the hydrolysis are water-miscible solvents such as lower alcohols, e.g., methanol or ethanol, glycols, e.g. propylene glycol, and lower ketones, e.g., acetone, as well as water itself.

Basic hydrolysis has the advantage in the process of the invention of yielding the product of the hydrolysis in the form of a salt which is readily separated from an organic phase composed of the starting material ester. Acidic hydrolysis, i.e., acid-catalyzed hydrolysis, may be advantageous if any of the substituents on the cyclopropane ring are base-sensitive. Acid-catalysed hydrolysis may be controlled, as the basic hydrolysis, to result in less than 100% conversion to the carboxylic acid with a difference in the cis/trans distribution between the starting ester and the carboxylic acid product.

The second step of either embodiment of the invention is the separation of the vicinal-substituted cyclopropane carboxylic acid product from the starting material. Conventional techniques such as crystallization, distillation, chromatography or separation by differences in solvent solubility may be used although the prefered method in view of simplicity is separation by solvent solubility. Thus, a water-immiscible solvent or solvents can be added to the reaction mixture to produce a two-phase system with the acid or salt product being more soluble in the water-miscible phase and the carboxylate starting material being more soluble in the water-miscible phase. Particular water-immiscible solvents include aromatic solvents such as benzene, toluene or xylene, higher ketones such as methyl ethyl ketone and methyl iso-butyl ketone, chlorinated solvents such as methylene chloride and chlorobenzene and esters such as ethyl acetate and butyl acetate. The two-phase system is shaken to achieve proper separation of the components between the two solvent system. Further, a phase separation aid such as an aqueous solution of sodium chloride or brine, may be added.

In the third step of the two embodiments of the invention, the cis-enriched carboxylate or trans-enriched acid or salt product is recovered, the enrichment being relative to the cis/trans distribution of the carboxylate before the hydrolysis reaction. If a two-phase solvent system is used for the second step separation, the recovery can be accomplished by drawing off or decanting one of the two phases.

In order to describe the invention so that it may be more clearly understood, the following examples are set forth. Abbreviations used include: °C (degrees centigrade); g (grams); ml (milliliters); mm Hg (milliliters of mercury pressure); C, H, N, O, Na, etc. (the universally-accepted symbols for the chemical elements); PAM (methyl-3-(2,2-dichlorovinyl)-2,2-dimethylcyclopropane carboxylate also known as permethrin acid methyl ester); and PAE (ethyl-3-(2,2-dichlorovinyl)-2,2-dimethylcyclopropane carboxylate also known as permethrin acid ethyl ester). All cis or trans contents are reported on a normalized basis, i.e., the amount of cis and trans isomer of the chemical entity being considered is considered to be 100%. The actual analysis may be different on a weight or molar basis since some inert or side reaction product may be present. Unless otherwise noted, all cis/trans percentage distributions are on a weight basis and determined percentage distributions are on a weight basis and determined by gas-liquid chromatography. The brine solution used in the Examples, unless otherwise noted, is an about 10% by weight sodium chloride in water solution obtained by mixing 100 g of NaCl in 1 liter of water.

Example 1

A mixture of 10 g of PAM having a 43/57 cis/trans distribution, 15 ml of methanol and a solution of 0.9 g of NaOH in 10 ml of water was heated with stirring at 64—67°C for 2 hours. In preparing this and other mixtures in the Examples, care should be taken to avoid contacting the cyclopropane carboxylate ester, such as PAM, directly with concentrated base. Thus, in the Example, the aqueous caustic was first diluted with the methanol after which the PAM was added. The NaOH represented about one half of the calculated amount necessary to fully hydrolyze the PAM. After cooling to room temperature, 20 ml of toluene and 40 ml of brine, were added and the mixture was shaken. The toluene layer was withdrawn and the solvents evaporated on a rotary vacuum evaporator to yield 6.0 g of unhydrolyzed PAM having a cis/trans distribution of about 54/46.

Example 2

To a solution of 20 g PAM having a 47.3/52.7 cis/trans distribution in 30 ml of methanol was added a solution of 1.8 g of NaOH in 20 ml water. The mixture was stirred and heated to 67°C for 2.5 hours. After cooling to room temperature, 40 ml of toluene and 80 ml of a sodium chloride brine solution were added and the mixture was shaken. After being allowed to settle, separation of the phases occurred and the toluene layer was withdrawn and vacuum evaporated at 60°C and 45 mm Hg followed by less than 1 mm Hg. Analysis of the 10.3 g of residue showed a 60.2/39.8 cis/trans distribution.

Example 3

A solution of 21 g PAE having a 42.9/57.1 cis/trans distribution in 30 ml of methanol was mixed at 67°C for 2.5 hours with a solution of 1.8 g NaOH in 20 ml of water. The organic layer was isolated as in Examples 1 and 2 using 40 ml of toluene and 80 ml of a brine solution. The 11.9 g of cis-enriched ester was found to be a mixture of PAM and PAE having a 53.4% cis content by weight. The molar ratio in the product of PAM:PAE was 0.7:1.

Example 4

The procedure and material amounts of Example 3 were repeated using 30 ml of ethanol in the place of methanol. The yield of cis-enriched PAE was 12.4 g which showed a 51.2% cis content.

Example 5

A solution of a 10 g of PAM having a 41.5/58.5 cis/trans distribution in 15 ml of methanol was mixed with a solution of 0.75 g NaOH in 10 ml of water and heated at 67°C for 1.5 hours. The reaction product was cooled to room temperature and 20 ml of toluene and 40 ml of brine were added. The mixture was shaken and the toluene layer was removed after phase separation. Evaporation of the toluene layer at 65°C and 10—20 mm Hg gave a product residue of 6.8 g. Analysis showed a 49.8/50.2 cis/trans distribution.

Example 6

The procedures of Example 5 were repeated using twice the amounts of each material with the exception of using 1.1 g of NaOH. After work-up, analysis of the 14.2 g residue showed a 47.5/52.5 cis/trans distribution.

Example 7

A solution of 20 g PAM having a 41.5/58.5 cis/trans distribution in 30 ml of methanol was mixed with a solution of 5.93 g potassium carbonate in 20 ml water and heated at 65—75°C for 2.5 hours. The reaction mixture was cooled and phase separation occurred after adding 40 g of toluene and 80 ml of brine and shaking. The toluene phase was drawn off and evaporated at 60°C and 40 mm Hg, followed by 5—7 mm Hg. Analysis of the 15.6 g of residue showed a 45/55 cis/trans distribution.

Example 8

A sample of 20 g of PAM having a 55.3/44.7 cis/trans distribution was treated in the manner described in Example 2 for 1.2 hours and worked up similarly. Analysis of the 10.7 g of residue showed a 70/30 cis/trans distribution.

Example 9

This example was carried out following the procedures and material amounts described in Example 2 except that the PAM had a 55.3/44.7 cis/trans distribution, the reaction time was 1.5 hours and 2.35 g of NaOH were used as opposed to 1.8 g. After the work-up described in Example 2, analysis of the 8.3 g of residue showed a 74.6/25.4 cis/trans distribution.

**Claims**

1. A method of enriching the cis content of a cis and trans isomeric mixture of a vicinal-substituted cyclopropane carboxylate of the formula:

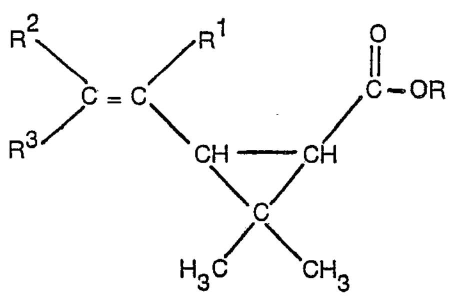

(IV)

wherein $R^1$ represents hydrogen, $R^2$ and $R^3$ independently represent halogen or a halogenated alkyl group; and R may be an alkyl, aryl, benzyl or substituted benzyl groups, which comprises the steps of:

i) partially hydrolyzing said vicinal-substituted cyclopropane carboxylate to form the corresponding vicinal-substituted cyclopropane carboxylic acid or salt;

ii) separating the vicinal-substituted cyclopropane carboxylic acid or salt formed in step i) from the unreacted vicinal-substituted cyclopropane carboxylate.

iii) recovering the cis-enriched vicinal-substituted cyclopropane carboxylate.

2. The method of Claim 1, wherein said partial hydrolysis step i) is to the extent of about 30 to 70 mole % of the vicinal-substituted cyclopropane carboxylate.

3. The method of Claim 1, wherein said partial hydrolysis step i) is at a temperature of less than about 100°C.

4. The method of Claim 1, wherein in said separation step ii), the vicinal-substituted cyclopropane carboxylic acid or salt is taken up in a water-miscible solvent and the vicinal-cyclopropane carboxylate is taken up in a water-immiscible solvent.

5. The method of Claim 1, wherein $R^1$ is hydrogen; $R^2$ and $R^3$ are halogen; and R is an alkyl or aryl group.

6. The method of Claim 5, wherein $R^2$ and $R^3$ are chlorine; and R is a lower alkyl group.

7. The method of Claim 6, wherein R is methyl or ethyl.

8. The method of anyone of Claims, 1, 2, 3, 4, 5, 6 or 7, wherein said hydrolyzing step i) is carried out under basic conditions.

9. A method of enriching the trans content of a cis and trans isomeric mixture of a vicinal-substituted cyclopropane carboxylic acid or salt compared to the trans content in a corresponding vicinal-substituted cyclopropane carboxylate comprising the steps of:

i) partially hydrolyzing said vicinal-substituted cyclopropane carboxylate to form the corresponding vicinal-substituted cyclopropane carboxylic acid or salt;

ii) separating the vicinal-substituted cyclopropane carboxylic acid or salt formed in step i) from the unreacted vicinal-substituted cyclopropane carboxylate.

iii) recovering the trans-enriched vicinal-substituted cyclopropane carboxylic acid.

10. The method of Claim 1, wherein said hydrolyzing step i) is carried out under basic conditions in the presence of an alkaline earth metal hydroxide or carbonate.

11. The method of Claim 1, wherein said hydrolyzing step i) is carried out under acidic conditions.

**Patentansprüche**

1. Verfahren zur Erhöhung des Gehalts an cis-Isomer eines Gemischs aus cis- und trans-Isomeren eines vicinal substituierten Cyclopropancarboxylats der Formel

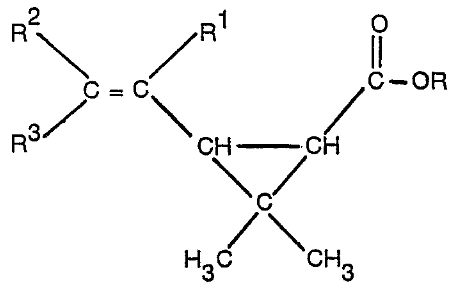

(IV)

worin $R^1$ für Wasserstoff steht, $R^2$ und $R^3$ unabhängig für Halogen oder eine halogenierte Alkylgruppe stehen und R für eine Alkyl-, Aryl-, Benzyl- oder substituierte Benzylgruppe steht, bei welchem

i) das vicinal substituierte Cyclopropancarboxylat teilweise hydrolysiert wird, um die entsprechende vicinal substituierte Cyclopropancarbonsäure oder ein Salz davon herzustellen,

ii) die in Stufe i) gebildete vicinal substituierten Cyclopropancarbonsäure oder das Salz davon vom nichtumgesetzten vicinal substituierten Cyclopropancarboxylat abgetrennt wird und

iii) das an cis-Isomer angereicherte vicinal substituierten Cyclopropancarboxylat gewonnen wird.

2. Verfahren nach Anspruch 1, bei welchem die teilweise Hydrolysestufe i) bis zu einem Ausmaß von ungefähr 30 bis 70 Mol-% des vicinal substituierten Cyclopropancarboxylats erfolgt.

3. Verfahren nach Anspruch 1, bei welchem die teilweise Hydrolysestufe i) bei einer Temperatur von weniger als ungefähr 100°C erfolgt.

4. Verfahren nach Anspruch 1, bei welchem in der Trennungsstufe ii) die vicinal substituierten Cyclopropancarbonsäure oder das Salz davon in einem mit Wasser mischbaren Lösungsmittel aufgenommen wird und das vicinale Cyclopropancarboxylat in einem mit Wasser unmischbaren Lösungsmittel aufgenommen wird.

5. Verfahren nach Anspruch 1, bei welchem $R^1$ für Wasserstoff steht, $R^2$ und $R^3$ für Halogen stehen und R für eine Alkyl- oder Arylgruppe steht.

6. Verfahren nach Anspruch 5, bei welchem $R^2$ und $R^3$ für Chlor stehen und R für eine Niederalkylgruppe steht.

7. Verfahren nach Anspruch 6, bei welchem R für Methyl oder Äthyl steht.

8. Verfahren nach einem der Ansprüche 1, 2, 3, 4, 5, 6 oder 7, bei welchem die Hydrolysestufe i) unter basischen Bedingungen ausgeführt wird.

9. Verfahren zur Erhöhung des Gehalts an trans-Isomer eines Gemischs aus cis- und trans-Isomeren einer vicinal substituierten Cyclopropancarbonsäure oder eines Salzes davon im Vergleich zum Gehalt an trans-Isomer in einem entsprechenden vicinal substituierten Cyclopropancarboxylat, bei welchem

i) das vicinal substituierten Cyclopropancarboxylat teilweise hydrolysiert wird, um die entsprechende vicinal substituierte Cyclopropancarbonsäure oder ein Salz davon herzustellen,

ii) die in Stufe i) gebildete vicinal substituierten Cyclopropancarbonsäure oder das Salz davon vom nichtumgesetzten vicinal substituierten Cyclopropancarboxylat abgetrennt wird und

iii) die an trans-Isomer angereicherte vicinal substituierte Cyclopropancarbonsäure gewonnen wird.

10. Verfahren nach Anspruch 1, bei welchem die Hydrolysestufe i) unter basischen Bedingungen in Gegenwart eines Erdalkalimetallhydroxids oder -carbonats ausgeführt wird.

11. Verfahren nach Anspruch 1, bei welchem die Hydrolysestufe i) unter sauren Bedingungen ausgeführt wird.

**Revendications**

1. Procédé pour accroître la teneur en isomère cis d'un mélange isomérique cis et trans d'un cyclopropanecarboxylate à substitution vicinale, de formule:

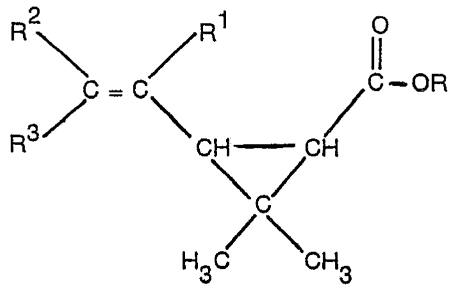

(IV)

dans laquelle $R^1$ représente l'hydrogène, $R^2$ et $R^3$ représentent, indépendamment, un halogène ou un groupe alkyle halogéné; et R peut être un groupe alkyle, aryle, benzyle ou benzyle substitué, qui comprend les étapes:

i) d'hydrolyse partielle dudit cyclopropanecarboxylate à substitution vicinale pour former l'acide cyclopropane-carboxylique à substitution vicinale correspondant ou un sel de cet acide;

ii) de séparation de l'acide cyclopropanecarboxylate à substitution vicinale ou de son sel formé dans l'étape i), du cyclopropane-carboxylate à substitution vicinale n'ayant pas réagi;

iii) d'isolement du cyclopropane-carboxylate à substitution vicinale enrichi en l'isomère cis.

2. Procédé suivant la revendication 1, dans lequel ladite étape i) d'hydrolyse partielle atteint la proportion d'environ 30 à 70 moles % du cyclopropanecarboxylate à substitution vicinale.

3. Procédé suivant la revendication 1, dans lequel ladite étape d'hydrolyse partielle i) est effectuée à une température inférieure à environ 100°C.

4. Procédé suivant la revendication 1, dans lequel l'acide cyclopropane-carboxylique à substitution vicinale ou son sel dans ladite étape de séparation ii) est repris dans un solvant miscible à l'eau et le cyclopropane-carboxylate vicinal est repris dans un solvant non miscible à l'eau.

5. Procédé suivant la revendication 1, dans lequel $R^1$ est l'hydrogène; $R^2$ et $R^3$ sont un halogène; et R est un groupe alkyle ou aryle.

6. Procédé suivant la revendication 5, dans lequel $R^2$ et $R^3$ sont du chlore; et R est un groupe alkyle inférieur.

7. Procédé suivant la revendication 6, dans lequel R est un groupe méthyle ou éthyle.

8. Procédé suivant l'une quelconque des revendications 1, 2, 3, 4, 5, 6 ou 7, dans lequel ladite étape d'hydrolyse i) est conduite dans des conditions basiques.

9. Procédé pour accroître la teneur en isomère rans d'un mélange isomérique cis et trans d'un acide cyclopropane-carboxylique à substitution vicinale ou d'un sel de cet acide, comparativement à la teneur en isomère trans d'un cyclopropane-carboxylate à substitution vicinale correspondant, comprenant les étapes:

i) d'hydrolyse partielle dudit cyclopropanecarboxylate à substitution vicinale pour former l'acide cyclopropane-carboxylique à substitution vicinale correspondant ou un sel de cet acide;

ii) de séparation de l'acide cyclopropanecarboxylate à substitution vicinale ou de son sel formé dans l'étape i) du cyclopropane-carboxylate à substitution vicinale n'ayant pas réagi;

iii) d'isolement de l'acide cyclopropane-carboxylique à substitution vicinale enrichi en isomère trans.

10. Procédé suivant la revendication 1, dans lequel ladite étape d'hydrolyse i) est conduite dans des conditions basiques en présence d'un hydroxyde ou d'un carbonate de métal alcalino-terreux.

11. Procédé suivant la revendication 1, dans lequel ladite étape d'hydrolyse i) est conduite dans des conditions acides.